# EUROPÄISCHE PATENTANMELDUNG

(11) **EP 3 260 164 A1**
(43) Veröffentlichungstag der Anmeldung: **27.12.2017**
(21) Anmeldenummer: 17182247.1
(22) Anmeldetag: 19.05.2015
(51) Int. Cl.: A61N 1/05, A61N 1/375, A61N 1/372, A61B 5/00, A61F 2/02

(54) **EINFÜHRVORRICHTUNG FÜR EIN IMPLANTAT**

(30) Priorität: 19.08.2014 US 201462038844 P
(62) Teilanmeldung aus: 15168075.8
(71) Anmelder: BIOTRONIK SE & Co. KG, 12359 Berlin (DE)
(72) Erfinder: DÖRR, Thomas, 12437 Berlin (DE); DIEBOLD, Michael, 12169 Berlin (DE)
(74) Vertreter: Keck, Hans-Georg

(57) **Zusammenfassung**

Die Erfindung betrifft eine Einführvorrichtung (200) geeignet für ein Implantat (100) das zum Einführen in einen menschlichen und/oder tierischen Körper geeignet ist und das ein Gehäuse (102) sowie zumindest eine Unterdruckeinheit (130) und zumindest eine Klebstoffapplikationseinheit (140) zur temporären und/oder permanenten Fixierung des Implantats (100) an einem Körpergewebe (110) umfasst, wobei das Implantat (100) mittels Unterdruck an der Einführvorrichtung (200) fixierbar ist und von der Einführvorrichtung (200) ablösbar ist, indem der Unterdruck auf die Unterdruckeinrichtung (130) des Implantats (100) umschaltbar ist.

## Beschreibung

Die Erfindung betrifft ein Implantat sowie eine Einführvorrichtung für ein Implantat nach den Oberbegriffen der unabhängigen Ansprüche.

Medizinische Implantate zum Einführen in den menschlichen oder tierischen Körper sind bekannt. Manche Implantate sind dazu vorgesehen, zu Diagnosezwecken und/oder zu Therapiezwecken temporär oder permanent an einem Körpergewebebereich fixiert zu werden.

Die US20060089690A1 beschreibt ein laparoskopisches Verfahren zur Befestigung eines solchen Implantates. Ein Teil des Körpergewebes wird mittels Vakuum in eine Kavität des Implantates gesaugt. Anschließend erfolgt eine mechanische Perforation und das Einbringen einer Fixiervorrichtung, womit eine dauerhafte Befestigung des Implantates am Körpergewebe realisiert wird. Eine Perforation an sich stellt eine Belastung des Gewebes dar und birgt Verletzungsrisiken.

Aus dem US-Patent US 6,689,056 B1 ist ein implantierbarer Sensor bekannt, der durch Unterdruck in einer Vertiefung auf der Sensoroberfläche oder durch Verkleben an einer Gewebeoberfläche befestigt werden kann. Darüber hinaus wird dort eine Vorrichtung zur Einführung des Sensors beschrieben.

Die US-Patentanmeldung US 20130013044 A1 beschreibt eine Elektrodenleitung zur Rückenmarksstimulation, deren Elektroden-Array mittels einer Klebevorrichtung mit dem zu stimulierenden Gewebe im Bereich des Rückenmarks verbunden werden kann. Zur Einbringung des Klebstoffs dient ein Kanal entlang der Elektrodenleitung. Dieser Kanal kann entweder an der Elektrodenleitung befestigt sein oder einen Bestandteil der Leitung darstellen.

Weiterhin beschreibt die US-Patentanmeldung US 20120041338 A1 eine Vorrichtung zur Abgabe oder Entnahme von Flüssigkeit wie z. B. Blut an die Haut oder darunterliegende Regionen. Diese Vorrichtung kann durch eine Klebeschicht an der Haut befestigt werden.

Der Erfindung liegt die Aufgabe zugrunde, ein Implantat anzugeben, das gewebeschonend an Körpergewebe temporär oder permanent befestigbar ist.

Eine weitere Aufgabe besteht in der Schaffung einer Einführvorrichtung für ein solches Implantat.

Die Aufgaben werden erfindungsgemäß durch die Merkmale der unabhängigen Ansprüche gelöst. Günstige Ausgestaltungen und Vorteile der Erfindung ergeben sich aus den weiteren Ansprüchen, der Beschreibung und der Zeichnung.

Es wird ein Implantat zum Einführen in einen menschlichen und/oder tierischen Körper vorgeschlagen, umfassend ein Gehäuse sowie zumindest eine Unterdruckeinheit und eine Klebstoffapplikationseinheit zur temporären und/oder permanenten Fixierung des Implantats an einem Körpergewebe.

Vorteilhaft kann das Implantat an einer Gewebeoberfläche positioniert und fixiert werden und zunächst temporär mittels Unterdruck und anschließend dauerhaft mittels eines Klebstoffs fixiert werden, ohne dabei die Gewebeoberfläche perforieren zu müssen. Das Implantat kann dabei auch zuverlässig auf einer nicht direkt sichtbaren Oberfläche und auf sich bewegenden Körpergeweben erfolgen. Vorteilhaft ist das Gebiet, in dem der Unterdruck angelegt wird, dasselbe Gebiet, in dem auch der Klebstoff appliziert wird.

Die Erfindung eignet sich für alle Implantate, die auf Körpergeweben fixiert werden sollen. Besonders bevorzugt ist der Klebstoff ein biokompatibler Klebstoff. Als biokompatibel werden Materialien verstanden, die entweder körpereigen sind oder welche nicht durch den menschlichen oder tierischen Organismus aufgenommen oder verstoffwechselt werden können.

Ein mit einer Gewebeperforation resultierendes Komplikationsrisiko kann vermieden werden. Insbesondere können Komplikationen wie ein Blutungs- und Infektionsrisiko und funktionelle Einschränkungen, wie Fibrosierung, Kinetikeinschränkungen oder ektopische Erregungen in Nerven- oder Myokardgewebe vermieden oder zumindest vermindert werden. Besonders günstig ist dies in Regionen mit starker Durchblutung oder bei Blutgefäßen, insbesondere dann, wenn bei der Implantation keine Sichtverbindung zum Implantationsort besteht.

Gemäß einer günstigen Ausgestaltung kann das Gehäuse an seiner bestimmungsgemäßen Kontaktseite mit dem Körpergewebe eine für eine Verklebung geeignete Klebefläche aufweisen. Insbesondere kann die Klebefläche eine Haftvermittlerstruktur, insbesondere eine poröse Struktur, aufweisen. Solche Strukturen, beispielsweise poröses Polyethylen, bewirken eine besonders gute Haftung des Klebstoffs.

Gemäß einer günstigen Ausgestaltung können an der Kontaktseite eine oder mehrere Dichtlippen zur lokalen Begrenzung einer Klebstoffausbreitung vorgesehen sein. Die Dichtlippen können das Ausbreitungsgebiet umschließen. Vorteilhaft kann der Klebstoff von Sensor- und/oder Elektrodenflächen am Implantat, die an der Kontaktseite des Implantats zum Körpergewebe vorgesehen sind, abgehalten werden. Gleichzeitig kann eine Dichtfunktion der Dichtlippen durch Anlegen von Unterdruck nachgeprüft werden, wenn die Gebiete, in denen Unterdruck und Klebstoff appliziert werden, überlappen.

Gemäß einer günstigen Ausgestaltung kann die Unterdruckeinheit als Vakuumschlauch ausgebildet sein. Insbesondere kann der Vakuumschlauch vom Implantat lösbar ausgebildet sein. Der Vakuumschlauch zum Applizieren des Unterdrucks kann auch einige Zeit nach dem Fixieren des Implantats im Körper verbleiben und dann später nichtinvasiv entfernt werden. Beispielsweise kann eine Entfernungshilfe angebracht sein, etwa ein Faden oder dergleichen, der von außen bedient werden kann.

Gemäß einer günstigen Ausgestaltung kann die Unterdruckeinheit als evakuierbares oder evakuiertes Unterdruckreservoir in das Implantat integriert sein. Das Unterdruckreservoir kann außerhalb des Körpers evakuiert werden und dann am Implantationsort geöffnet werden.

Gemäß einer günstigen Ausgestaltung kann die Unterdruckeinheit so mit der Klebstoffapplikationseinheit gekoppelt sein, dass ein Unterdruck gezielt eine Klebstofffreisetzung bewirkt. Die Klebstofffreisetzung kann durch den Unterdruck gestartet bzw. gesteuert werden, indem beispielsweise eine Verschlussmembran der Klebstoffapplikationseinheit sich ab einem definierten Unterdruck öffnet und den Klebstoff freigibt. Wird die Klebstoffverteilung durch den Unterdruck bewirkt, kann gleichzeitig am Druckverlauf und/oder am Volumenverlauf erkannt werden, ob der Klebeprozess abgeschlossen ist, beispielsweise wenn alle vorgesehenen Kanäle und/oder Freiräume mit Klebstoff gefüllt und verschlossen sind.

Gemäß einer günstigen Ausgestaltung kann die Klebstoffapplikationseinheit als Klebstoff oder als Ausgangsmaterial für Klebstoff wenigstens eine Komponente aus der Gruppe abgeben: Glykoprotein, insbesondere Fibrinogen als Glykoprotein, 2-Octyl-Cyanoacrylat, vernetzte Gelatine. Besonders vorteilhaft kann die von der die Klebstoffapplikationseinheit als Klebstoff oder als Ausgangsmaterial für Klebstoff applizierte Komponente eine Basisstruktur zur Bildung eines Thrombus sein.

Insbesondere kann der Klebstoff aus wenigstens zwei Hauptbestandteilen gebildet sein, wobei einer der Hauptbestandteile ein Glykoprotein, insbesondere Fibrinogen als Glykoprotein, und/oder ein Proteaseinhibitor, insbesondere Aprotinin als Proteaseinhibitor, und ein anderer der Hauptbestandteile Thrombin und/oder Calciumchlorid sein. Vorteilhaft kann der eine Hauptbestandteil des Klebstoffs eine Basisstruktur zur Bildung eines Thrombus sein und ein anderer Hauptbestandteil ein oder mehrere vernetzende Zusatzstoffe, wie z.B. Thrombin und/oder Faktor XIII. Faktor XIII ist, wie Fibrinogen selbst, ein Gerinnungsfaktor. Dieser wirkt in der Gerinnungskette nach dem Fibrinogen bzw. auf das umgewandelte Fibrinogen, Fibrin.

Gemäß einer günstigen Ausgestaltung kann der Klebstoff einen Fibrinklebeschaum umfassen. Dieser lässt sich besonders leicht applizieren.

Gemäß einer günstigen Ausgestaltung kann die Klebstoffapplikationseinheit ein Mischelement zum Mischen von Klebstoffkomponenten aufweisen. Bei der Verwendung eines Klebstoffs aus mehreren Bestandteilen, wie etwa ein Fibrinkleber, ist es vorteilhaft, im Implantat einen Mischer für die Bestandteile vorzusehen, so dass die Bestandteile erst kurz vor der Freisetzung gemischt werden. Dies erhöht die Zuverlässigkeit der Klebung.

Gemäß einer günstigen Ausgestaltung kann das Implantat als Pulsgenerator, Diagnostiksensor oder Elektrodenleitung ausgebildet sein. Generell ist die Erfindung für alle Implantate, die auf Körpergeweben fixiert werden sollen, geeignet.

Gemäß eines weiteren Aspekts der Erfindung wird eine Einführvorrichtung für ein Implantat vorgeschlagen, wobei das Implantat zum Einführen in einen menschlichen und/oder tierischen Körper vorgesehen ist und ein Gehäuse sowie zumindest eine Unterdruckeinheit und eine Klebstoffapplikationseinheit zur temporären und/oder permanenten Fixierung des Implantats an einem Körpergewebe umfasst. Dabei ist das Implantat mittels Unterdruck an der Einführvorrichtung fixierbar und von der Einführvorrichtung ablösbar, indem der Unterdruck auf die Unterdruckeinheit des Implantats umschaltbar ist. Der Unterdruck kann beispielsweise durch das Betätigen eines Ventils auf die Unterdruckeinheit des Implantates umleitbar sein.

Gemäß einer günstigen Ausgestaltung kann ein Unterdrucksensor vorgesehen sein. Dieser kann zur Erkennung der korrekten Platzierung des Implantats und zur Überprüfung der Klebung eingesetzt werden. So kann die Klebstofffreisetzung durch den Unterdruck gestartet bzw. gesteuert werden, indem beispielsweise eine Verschlussmembran der Klebstoffapplikationseinheit sich ab einem definierten Unterdruck öffnet und den Klebstoff freigibt. Wird die Klebstoffverteilung durch den Unterdruck bewirkt, kann gleichzeitig am Druckverlauf und/oder am Volumenverlauf erkannt werden, ob der Klebeprozess abgeschlossen ist, beispielsweise wenn alle vorgesehenen Kanäle und/oder Freiräume mit Klebstoff gefüllt und verschlossen sind.

Die Erfindung ist nachfolgend beispielhaft, anhand von in Zeichnungen dargestellten Ausführungsbeispielen, näher erläutert. Es zeigen in schematischer Darstellung:
- Fig. 1a, 1b: einen Schnitt durch eine Ausgestaltung eines Implantats vor der Fixierung am Körpergewebe mit einer Vakuumleitung (Figur 1a) und einem Unterdruckreservoir (Figur 1b);
- Fig. 2a-2c: in Schnittansicht drei Verfahrensschritte beim Fixieren eines Implantats;
- Fig. 3: eine alternative Vorrichtung zur Klebemittelapplikation;
- Fig. 4a, 4b: als Schnittansicht und in Draufsicht Dichtelemente eines Implantats, und
- Fig. 5: eine Einführvorrichtung mit einem Implantat nach einer Ausgestaltung der Erfindung.

In den Figuren sind funktionell gleiche oder gleich wirkende Elemente jeweils mit denselben Bezugszeichen beziffert. Die Figuren sind schematische Darstellungen der Erfindung. Sie bilden nicht spezifische Parameter der Erfindung ab. Weiterhin geben die Figuren lediglich typische Ausgestaltungen der Erfindung wieder und sollen die Erfindung nicht auf die dargestellten Ausgestaltungen beschränken.

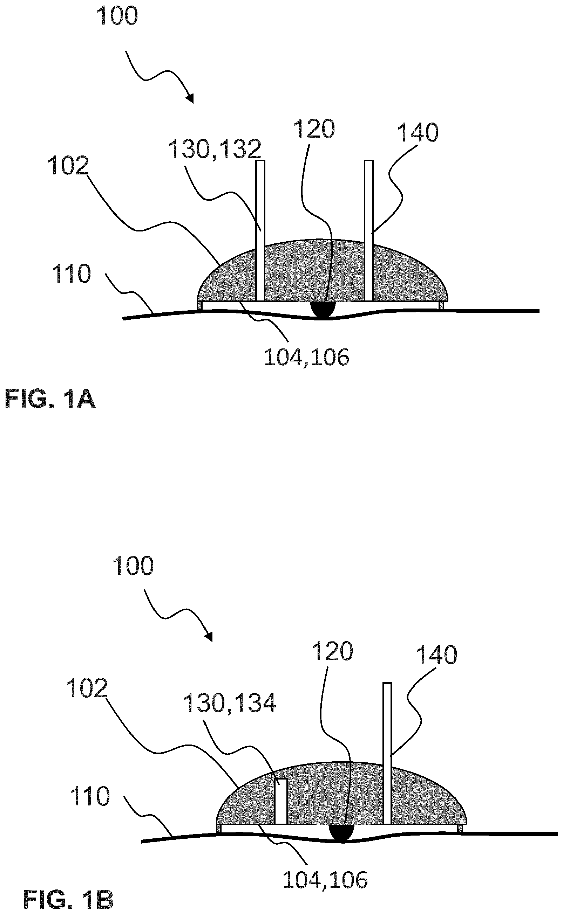

Die Figuren 1a und 1b zeigen zur Erläuterung der Erfindung jeweils eine Ausgestaltung eines Implantats 100 zum Einführen in einen menschlichen und/oder tierischen Körper, umfassend ein Gehäuse 102 sowie zumindest eine Unterdruckeinheit 130 und zumindest eine Klebstoffapplikationseinheit 140 zur temporären und/oder permanenten Fixierung des Implantats 100 an einem Körpergewebe 110. Das Implantat 100 ist bevorzugt ein dauerimplantierbares medizinisches Gerät, das zur Fixierung auf einem Körpergewebe bestimmt ist. Es umfasst mindestens die eine Unterdruckeinheit 130 zur temporären Fixierung am Körpergewebe, die durch einen eingebrachten Unterdruck das Implantat 100 an dem Körpergewebe festlegt und mindestens die eine Klebstoffapplikationseinheit 140 zur Applikation eines oder mehrerer biokompatibler Klebstoffe, die die Klebstoffapplikationseinheit 140 während der bestehenden Unterdruckfixierung gestattet.

Das Implantat weist am Gehäuse 102 mindestens einer Therapie- oder Sensoreinheit 120 auf, die im Kontakt mit dem Körpergewebe 110 stehen soll.

Die Gehäuseunterseite stellt die bestimmungsgemäße Kontaktseite 104 des Implantats 100 mit dem Körpergewebe 110 und weist eine für eine Verklebung geeignete Klebefläche 106 auf. Die Klebefläche 106 kann eine Haftvermittlerstruktur, insbesondere eine poröse Struktur, aufweisen, mit der sich der Klebstoff gut verbindet

Während der Unterdruckfixierung kann vorteilhaft eine temporäre Testung der Implantatfunktionen vorgesehen sein. Wird eine Fehlfunktion festgestellt, kann der Unterdruck entfernt und das Implantat 100 ersetzt werden. Stellt sich bei der Testung heraus, dass das Implantat 100 nicht korrekt platziert ist, kann dies durch Brechen des Unterdrucks, Verschieben des Implantats 100 und erneutes Anlegen des Unterdrucks behoben werden.

Im einfachsten Fall ist die Unterdruckeinheit 130 eine schlauchförmige Leitung 132 (Figur 1a), die in einer Kavität an der Gehäuseunterseite endet und an deren anderem Ende eine Vorrichtung zur Evakuierung vorhanden ist, im einfachsten Fall eine Spritze, oder auch eine geeignete Pumpe.

In Figur 1a ist schematisch eine Variante dargestellt, bei der statt des Vakuumschlauchs 132 ein Unterdruckreservoir 134 vorgesehen ist, das extern außerhalb des Körpers evakuiert wird und am Implantationsort geöffnet werden kann.

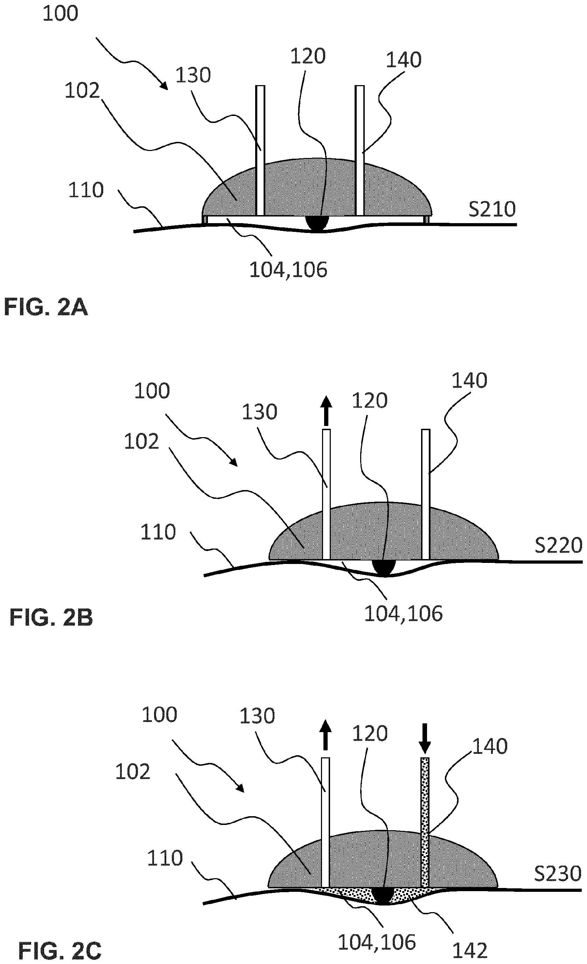

Die Figuren 2a-2c zeigen in Schnittansicht drei Verfahrensschritte beim Fixieren eines Implantats 100, wobei eine Einführeinrichtung nicht dargestellt ist.

Im ersten Schritt S210 wird das Implantat 100 auf dem Zielgewebe 110 mit Hilfe einer nicht dargestellten Einführhilfe platziert.

Im zweiten Schritt S220 wird ein Unterdruck an der Gehäuseunterseite (Kontaktseite 104) erzeugt und so das Implantat 100 temporär am Körpergewebe 110 fixiert. In diesem Zustand können die Implantatfunktionen getestet und somit die Eignung des Ortes der Implantation für den bestimmungsgemäßen Gebrauch des Implantats 100 überprüft werden. Sollte diese Überprüfung negativ ausfallen, kann durch Abbau des Unterdruckes das Implantat 100 rückstands- und verletzungsfrei von der Körpergewebeoberfläche wieder abgelöst und umpositioniert werden. Dies stellt einen Vorteil dieser Methode dar. Ferner kann die temporäre Testung von Implantatfunktionen in diesem Zustand auch ohne Vorhandensein der Einführhilfe durchgeführt werden, was z.B. bei Implantaten 100 auf sich bewegenden Körperoberflächen, wie zum Beispiel dem Myokard einen weiteren Vorteil darstellt bzw. derartige Tests erst ermöglicht.

Ist die Testung positiv verlaufen, wird in einem dritten Schritt S230 zur endgültigen Fixierung des Implantats 100 ein biokompatibler Klebstoff, wie z.B. Fibrinkleber, 2-Octyl-Cyanoacrylat oder ein alternativer Klebstoff, z.B. eine vernetzte Gelatine, wie z.B. LifeSeal der Firma Lifebond Israel, durch die Klebstoffapplikationseinrichtung 140 appliziert. Die Klebstoffapplikation erfolgt dabei immer unter Aufrechterhaltung des Unterdruckes im Bereich der Klebefläche 106. Damit kann auch die Ausbreitung des Klebstoffs gesteuert bzw. gefördert werden. Der Unterdruck bleibt zweckmäßigerweise so lange bestehen, bis eine ausreichende Aushärtung des Klebstoffs erfolgt ist.

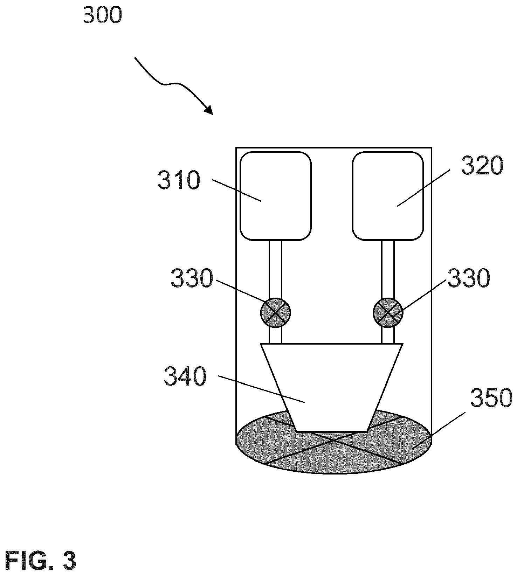

Figur 3 zeigt eine alternative Vorrichtung zur Klebemittelapplikation. Geeignete Fibrinklebemittel weisen vorwiegend zwei Hauptkomponenten auf, eine fibrinogenhaltige Komponente und eine thrombinhaltige Komponente, beide bei Verwendung in flüssiger Form.

Die geeigneten Komponenten des Fibrinklebemittels sind z.B. entweder Fibrinogen und Faktor XIII als erster Hauptbestandteil und Thrombin als zweiter Hauptbestandteil, oder Aprotinin als erster Hauptbestandteil und Calciumchlorid als zweiter Hauptbestandteil. Thrombin und Fibrinogen bewirken im vermischten Zustand die Aufspaltung der Peptidketten des Fibrinogens, so dass das resultierende Fibrin zu einem Koagulat bzw. Klumpen polymerisiert, das zur Verbindung von Implantatgewebe und Körpergewebe geeignet ist.

Um eine frühzeitige Koagulation zu vermeiden, dürfen die Bestandteile des Fibrinklebemittels erst am Wirkort vermischt werden, d.h. die beiden Bestandteile müssen bis zum eigentlichen Klebevorgang getrennt gehalten werden.

Die hier dargestellte Vorrichtung 300 weist daher mehrere, hier zwei, getrennte Klebstoffbestandteildepots 310, 320 mit zwei getrennten Zuleitungen für die Bestandteile des Fibrinklebemittels und eine Mischvorrichtung 340 zum Mischen von zwei oder mehr Bestandteilen im Implantat 100 auf.

Die Figur zeigt weiterhin zwei Freisetzungseinrichtungen 330 für die Klebstoffbestandteile des Fibrinklebers. Diese Freisetzungseinrichtungen sind hier z.B. derart konstruiert, dass diese sich bei einem definierten Druck öffnen, wobei dieser Druck durch die Erhöhung des Unterdrucks eingestellt wird, so dass der Klebeprozess vorteilhaft über die Steuerung des Unterdrucks auf sehr einfache Weise gesteuert werden kann.

Nach Freisetzung der Klebstoffbestandteile werden diese in der Mischvorrichtung 340 nach einem Verwirbelungsprinzip vermischt und dann durch ein poröses Polymerklebetarget 350 in die vom Unterdruck betroffene Kavität zwischen Implantat 100 und Körpergewebe 110 freigesetzt und damit das Implantat 100 an das Zielgewebe geklebt.

Die Vorrichtung eignet sich grundsätzlich für alle Kleberarten und ist bei Klebstoffen mit nur einem Bestandteil entsprechend einfacher aufgebaut.

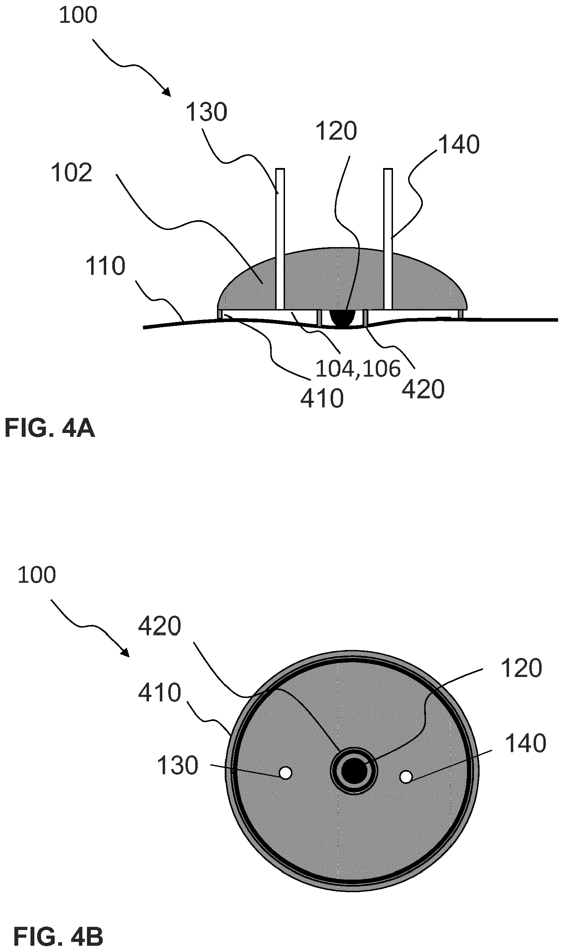

Die Figuren 4a und 4b zeigen als Schnittansicht (Figur 4a) und in Draufsicht (Figur 4b) ein Implantat mit Dichtelementen 410, 420, beispielsweise Dichtlippen oder Dichtrahmen. Das äußere Dichtelement 410 verhindert die Ausbreitung des Klebstoffs außerhalb des Implantats 100, während das innere Dichtelement 420 die Ausbreitung des Klebstoffs in den Bereich verhindert, in dem das Therapie- oder Sensorelement 120 angeordnet ist, so dass dessen Funktion vom Klebstoff ungestört bleibt. Der Klebstoff kann sich nur innerhalb des von den Dichtelementen 410, 420 begrenzten Bereichs ausbreiten.

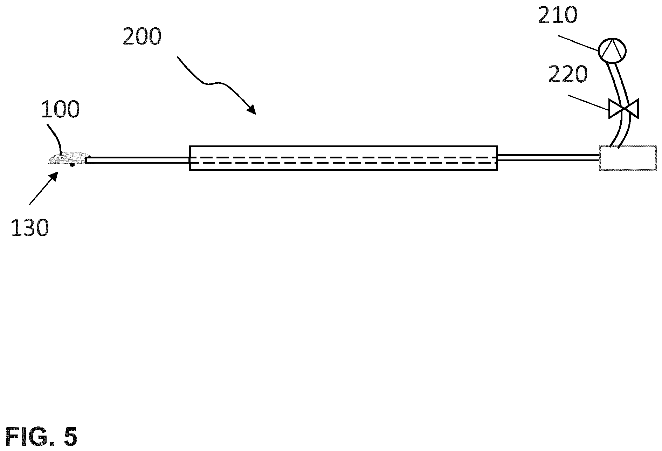

Figur 5 zeigt vereinfacht eine Einführvorrichtung 200, z.B. ein Laparoskop, ein Katheter oder dergleichen, mit einem Implantat 100 nach einer Ausgestaltung der Erfindung.

Beim Einführen des Implantats 100 ist dieses mittels Unterdruck an der Einführvorrichtung 200 gehalten. Der Unterdruck wird von einer externen Einheit 210, z.B. einer Pumpe oder dergleichen, bereitgestellt. Die Unterdruckeinheit 130 im Implantat 100 ist ebenfalls mit der Einheit 210 verbunden. Ein Ventil 220 am proximalen Ende der Einführvorrichtung 200 ist vorgesehen, um den Unterdruck zwischen einer Haltevorrichtung des Implantats 100 an der Einführvorrichtung 200 und der Unterdruckeinheit 130 im Implantat umzuschalten. Dabei löst sich das Implantat 100 von der Einführvorrichtung 200 und saugt sich am Körpergewebe fest.

Die Erfindung ermöglicht es, ein Implantat 100 an einer Gewebeoberfläche 110 ohne deren Perforation temporär und dauerhaft zu fixieren und erlaubt damit die Positionierung von Implantaten 100 an derzeit unzugänglichen Implantationsorten.

## Patentansprüche

1. Einführvorrichtung (200) geeignet für ein Implantat (100) das zum Einführen in einen menschlichen und/oder tierischen Körper geeignet ist und das ein Gehäuse (102) sowie zumindest eine Unterdruckeinheit (130) und zumindest eine Klebstoffapplikationseinheit (140) zur temporären und/oder permanenten Fixierung des Implantats (100) an einem Körpergewebe (110) umfasst,
**dadurch gekennzeichnet, dass**
das Implantat (100) mittels Unterdruck an der Einführvorrichtung (200) fixierbar ist und von der Einführvorrichtung (200) ablösbar ist, indem der Unterdruck auf die Unterdruckeinrichtung (130) des Implantats (100) umschaltbar ist.

2. Einführvorrichtung nach Anspruch 1, die in Form eines Laparoskops oder eines Katheters ausgeführt ist.

3. Einführvorrichtung gemäß einer der vorstehenden Ansprüche, wobei der Unterdruck von einer externen Einheit (210) bereitgestellt wird, die nicht Bestandteil der Einführvorrichtung (200) ist.

4. Einführvorrichtung gemäß einer der vorstehenden Ansprüche, wobei der Unterdruck durch das Betätigen eines Ventils (220) auf die Unterdruckeinheit (130) des Implantates (100) umleitbar ist.

5. Einführvorrichtung gemäß einer der vorstehenden Ansprüche, wobei die Klebstofffreisetzung durch den Unterdruck gestartet und/oder gesteuert wird.

6. Einführvorrichtung nach Anspruch 5, wobei sich eine Verschlussmembran der Klebstoffapplikationseinheit (140) ab einem definierten Unterdruck öffnet und den Klebstoff freigibt.

7. Einführvorrichtung gemäß einer der vorstehenden Ansprüche, wobei ein Unterdrucksensor (202) vorgesehen ist.
